(19) Europäisches Patentamt
European Patent Office
Office européen des brevets

(11) EP 2 014 636 A1

# (12) EUROPEAN PATENT APPLICATION

(43) Date of publication:
**14.01.2009 Bulletin 2009/03**

(51) Int Cl.:
***C07C 13/615*** *(2006.01)* ***C07C 25/02*** *(2006.01)*

(21) Application number: **08011056.2**

(22) Date of filing: **18.06.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **05.07.2007 JP 2007176956**

(71) Applicant: **Daicel Chemical Industries, Ltd.**
**Kita-ku,**
**Osaka-shi**
**Osaka 530-0001 (JP)**

(72) Inventors:
- **Yasuhito, Nakai**
**Himeji-shi**
**Hyogo 671-1283 (JP)**
- **Shigehiro, Maeda**
**Tokyo 108-8230 (JP)**

(74) Representative: **Grünecker, Kinkeldey, Stockmair & Schwanhäusser**
**Anwaltssozietät**
**Leopoldstrasse 4**
**80802 München (DE)**

## (54) Ethynylphenylbiadamantane derivatives

(57) Disclosed is a halophenylbiadamantane derivative of following Formula (1):

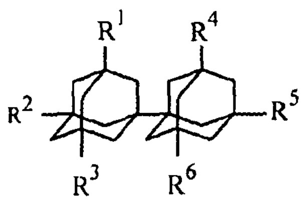

(1)

wherein $R^1$, $R^2$, $R^3$, $R^4$, $R^5$, and $R^6$ each independently represent, for example, a hydrogen atom or a halo-substituted or unsubstituted alkyl or cycloalkyl group having about six or less carbon atom(s), provided that at least one of $R^1$ to $R^6$ represents a substituent represented by following Formula (2):

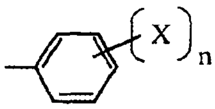

(2)

wherein X represents a halogen atom; and "n" denotes a natural number of 1 to 5, wherein when only one of $R^1$ to $R^6$ is a substituent of Formula (2), then at least one of $R^1$ to $R^6$ is a halo-substituted or unsubstituted alkyl or cycloalkyl group having about six or less carbon atom(s) or "n" is a natural number of 2 or more. The halophenylbiadamantane derivative is usable as an intermediate material for a wide variety of biadamantane derivatives.

EP 2 014 636 A1

**Description**

BACKGROUND OF THE INVENTION

1. Field of the Invention

The present invention relates to novel ethynylphenylbiadamantane derivatives that can be used typically as materials for interlayer dielectric films of multilayer circuit boards, materials for flexible printed circuit boards, and materials for aerospace use. It also relates to novel halophenylbiadamantane derivatives that can be used as starting materials for a variety of adamantane derivatives, and processes for the preparation of the ethynylphenylbiadamantane derivatives.

2. Description of the Related Art

Adamantane derivatives have stable carbon skeleton structures, thereby have satisfactory properties such as thermal stability, water resistance, optical properties, optical transparency, low dielectric constant, and stiffness (rigidity), and have been used as starting materials to give functional materials, such as high-functional polymers, which excel typically in thermal properties, mechanical properties, electrical properties, and/or optical properties. Among such adamantane derivatives, biadamantane derivatives each having two adamantane skeletons bonded to each other further excel typically in thermal properties as compared to adamantane and diadamantane derivatives and are expected to be applied as starting materials to give high-functional materials such as materials for electronic components and optical components.

Additionally, there have been known techniques of forming a polymer network to improve mechanical strength, by incorporating a crosslinkable functional group into a monomer component or directly into a polymer; and there have been proposed the application of an adamantane derivative (Japanese Unexamined Patent Application Publication (JP-A) No. 2003-292878), a diadamantane derivative (JP-A No. 2006-257212), and a biadamantane derivative (JP-A No. 2005-516382) each having an ethynyl group, to give semiconductor devices, such as porous dielectric films, which excel in dielectric constant, mechanical strength, and thermal stability.

In this connection, the solubility of monomers in organic solvents is an important factor upon preparation of polymers containing adamantane skeletons. It is therefore desirable to provide ethynyl-containing biadamantane derivatives that excel in dielectric constant, mechanical strength, and thermal stability, have higher solubility in organic solvents, and are advantageous in production and processing.

On the other hand, halophenylbiadamantane derivatives can be widely utilized as intermediate materials and are very useful, because their halogen atom can be converted into a variety of substituents such as carbonyl group, an alkyl group, phenyl group, an alkenyl group, or alkynyl group (e.g., ethynyl group). However, there has been disclosed no halophenylbiadamantane derivative but 3-(4-bromophenyl)-1,1'-biadamantane as a material for 1-(4-vinylphenyl) adamantane (JP-A No. 2006-96988) alone.

SUMMARY OF THE INVENTION

Accordingly, an object of the present invention is to provide a novel ethynylphenylbiadamantane derivative that is useful as a functional material excellent typically in electrical properties, thermal properties, mechanical properties, and/or physical properties.

Another object of the present invention is to provide a novel halophenylbiadamantane derivative that is an intermediate material for the ethynylphenylbiadamantane derivative and is widely usable as an intermediate material for a variety of adamantane derivatives.

Yet another object of the present invention is to provide a process for the preparation of the ethynylphenylbiadamantane derivative from the halophenylbiadamantane derivative.

After intensive investigations to achieve the above objects, the present inventors found a novel ethynylphenylbiadamantane derivative that has higher crosslinkability and higher solubility in solvents than the known ethynylphenylbiadamantane derivative having a monoethynylphenyl group.

They also found a novel halophenylbiadamantane derivative that is an intermediate material for the ethynylphenylbiadamantane derivative and, in addition, is usable as an intermediate material for a wide variety of biadamantane derivatives.

They further found a process for efficiently preparing the ethynylphenylbiadamantane derivative from the halophenylbiadamantane derivative. Additionally, they also found a process for deprotecting (removing a protecting group of) the ethynylphenylbiadamantane derivative. The present invention has been made based on these findings.

Specifically, according to an embodiment of the present invention, there is provided a halophenylbiadamantane derivative which is represented by following Formula (1):

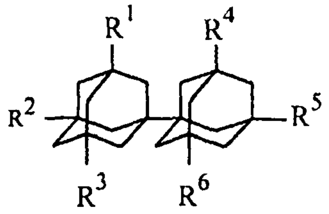

(1)

wherein $R^1$, $R^2$, $R^3$, $R^4$, $R^5$, and $R^6$ are the same as or different from one another and each represent a hydrogen atom, a halo-substituted or unsubstituted alkyl or cycloalkyl group having about six or less carbon atom(s), a substituted or unsubstituted phenyl group, a carboxyl group, a substituted or unsubstituted carbamoyl group, a substituted oxycarbonyl group, a substituted or unsubstituted amino group, a halogen atom, or a hydroxyl group, with the proviso that at least one of $R^1$ to $R^6$ represents a substituent represented by following Formula (2) :

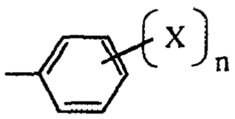

(2)

wherein X represents a halogen atom; and "n" denotes a natural number of 1 to 5, wherein when two or more of $R^1$ to $R^6$ represent substituents of Formula (2), these substituents may be the same as or different from one another, wherein the benzene ring in Formula (2) may further have an alkyl or cycloalkyl group having about six or less carbon atoms in addition to the halogen atom(s), and wherein when only one of $R^1$ to $R^6$ is a substituent of Formula (2), then at least one of $R^1$ to $R^6$ is a halo-substituted or unsubstituted alkyl or cycloalkyl group having about six or less carbon atom(s) or "n" is a natural number of 2 or more.

Such novel halophenylbiadamantane derivatives are widely usable as materials for a wide variety of adamantane derivative.

According to another embodiment of the present invention, there is provided an ethynylphenylbiadamantane derivative which is represented by following Formula (3):

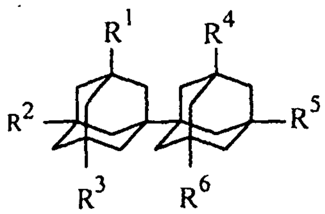

(3)

wherein $R^1$, $R^2$, $R^3$, $R^4$, $R^5$, and $R^6$ are the same as or different from one another and each represent a hydrogen atom, a halo-substituted or unsubstituted alkyl or cycloalkyl group having about six or less carbon atom(s), a substituted or unsubstituted phenyl group, a carboxyl group, a substituted or unsubstituted carbamoyl group, a substituted oxycarbonyl group, a substituted or unsubstituted amino group, a halogen atom, or a hydroxyl group, with the proviso that at least one of $R^1$ to $R^6$ represents a substituent represented by following Formula (4) :

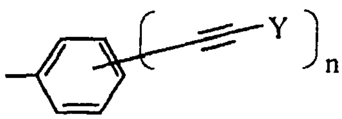

(4)

wherein Y represents a hydrogen atom, an alkyl group having about one to about six carbon atoms, a phenyl group, or a tri-substituted silyl group; and "n" denotes a natural number of 1 to 5, wherein when two or more of $R^1$ to $R^6$ represent substituents of Formula (4), these substituents may be the same as or different from one another, and wherein when "n" is 1 in all the substituent(s) of Formula (4) as $R^1$ to $R^6$, then at least one of $R^1$ to $R^6$ is a halo-substituted or unsubstituted alkyl or cycloalkyl group having about six or less carbon atom(s).

Such novel ethynylphenylbiadamantane derivatives are satisfactorily soluble in solvents and are usable as starting materials to give functional materials excellent typically in electrical properties, thermal properties, mechanical properties, optical properties, and/or physical properties.

According to yet another embodiment of the present invention, there is provided a process for the preparation of ethynylphenylbiadamantane derivatives. The process includes the step of reacting a halophenyladamantane derivative with a terminal alkyne to give an ethynylphenylbiadamantane derivative,
in which the halophenyladamantane derivative is represented by following Formula (1a):

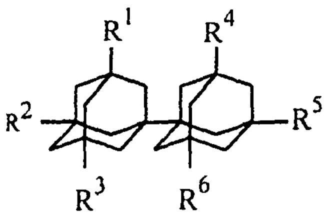

(1 a)

wherein $R^1$, $R^2$, $R^3$, $R^4$, $R^5$, and $R^6$ are the same as or different from one another and each represent a hydrogen atom, a halo-substituted or unsubstituted alkyl or cycloalkyl group having about six or less carbon atom(s), a substituted or unsubstituted phenyl group, a carboxyl group, a substituted or unsubstituted carbamoyl group, a substituted oxycarbonyl group, a substituted or unsubstituted amino group, a halogen atom, or a hydroxyl group, with the proviso that at least one of $R^1$ to $R^6$ represents a substituent represented by following Formula (2) :

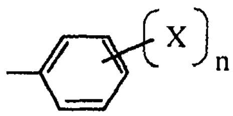

(2)

wherein X represents a halogen atom; and "n" denotes a natural number of 1 to 5, wherein, when two or more of $R^1$ to $R^6$ represent substituents of Formula (2), these substituents may be the same as or different from one another, wherein the benzene ring in Formula (2) may further have an alkyl or cycloalkyl group having about six or less carbon atoms in addition to the halogen atom(s), and wherein when "n" is 1 in all the substituent(s) of Formula (2) as $R^1$ to $R^6$, then at least one of $R^1$ to $R^6$ is a halo-substituted or unsubstituted alkyl or cycloalkyl group having about six or less carbon atom(s),
the terminal alykyne is represented by following Formula (5):

$$R'-C\equiv C-Y \qquad (5)$$

wherein Y represents a hydrogen atom, an alkyl group having about one to about six carbon atoms, a phenyl group, or a tri-substituted silyl group; and R' represents a hydrogen atom or a metal atom, and
the ethynylphenylbiadamantane derivative is represented by following Formula (3):

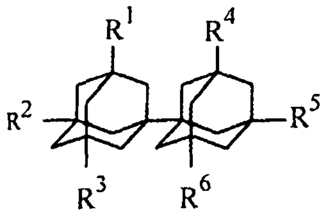

(3)

wherein $R^1$, $R^2$, $R^3$, $R^4$, $R^5$, and $R^6$ are the same as or different from one another and each represent a hydrogen atom, a halo-substituted or unsubstituted alkyl or cycloalkyl group having about six or less carbon atom(s), a substituted or unsubstituted phenyl group, a carboxyl group, a substituted or unsubstituted carbamoyl group, a substituted oxycarbonyl group, a substituted or unsubstituted amino group, a halogen atom, or a hydroxyl group, with the proviso that at least one of $R^1$ to $R^6$ represents a substituent represented by following Formula (4):

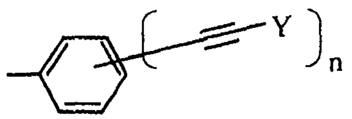

(4)

wherein Y represents a hydrogen atom, an alkyl group having about one to about six carbon atoms, a phenyl group, or a tri-substituted silyl group; and "n" denotes a natural number of 1 to 5, wherein when two or more of $R^1$ to $R^6$ represent substituents of Formula (4), these substituents may be the same as or different from one another, wherein when "n" is 1 in all the substituent(s) of Formula (4) as $R^1$ to $R^6$, then at least one of $R^1$ to $R^6$ is a halo-substituted or unsubstituted alkyl or cycloalkyl group having about six or less carbon atom(s).

In addition and advantageously, there is provided another process for the preparation of ethynylphenylbiadamantane derivatives. This process includes the step of reacting an ethynylphenylbiadamantane derivative (3a) with an alcohol compound to give another ethynylphenylbiadamantane derivative (3b),
in which the ethynylphenylbiadamantane derivative (3a) is represented by following Formula (3a):

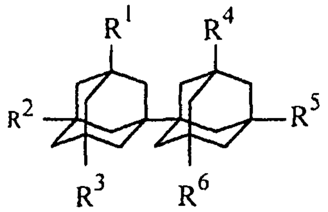

(3a)

wherein $R^1$, $R^2$, $R^3$, $R^4$, $R^5$, and $R^6$ are the same as or different from one another and each represent a hydrogen atom, a halo-substituted or unsubstituted alkyl or cycloalkyl group having about six or less carbon atom(s), a substituted or unsubstituted phenyl group, a carboxyl group, a substituted or unsubstituted carbamoyl group, a substituted oxycarbonyl group, a substituted or unsubstituted amino group, a halogen atom, or a hydroxyl group, with the proviso that at least one of $R^1$ to $R^6$ represents a substituent represented by following Formula (4a):

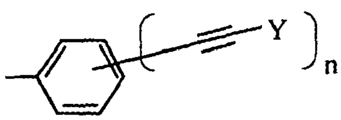

(4a)

wherein Y represents a tri-substituted silyl group; and "n" denotes a natural number of 1 to 5, wherein when two or more of $R^1$ to $R^6$ represent substituents of Formula (4a), these substituents may be the same as or different from one another,

wherein when "n" is 1 in all the substituent(s) of Formula (4a) as $R^1$ to $R^6$, then at least one of $R^1$ to $R^6$ is a halo-substituted or unsubstituted alkyl or cycloalkyl group having about six or less carbon atom(s) or and
the ethynylphenylbiadamantane derivative (3b) is represented by following Formula (3b):

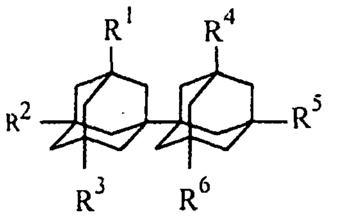

(3b)

wherein $R^1$, $R^2$, $R^3$, $R^4$, $R^5$, and $R^6$ are the same as or different from one another and each represent a hydrogen atom, a halo-substituted or unsubstituted alkyl or cycloalkyl group having about six or less carbon atom(s), a substituted or unsubstituted phenyl group, a carboxyl group, a substituted or unsubstituted carbamoyl group, a substituted oxycarbonyl group, a substituted or unsubstituted amino group, a halogen atom, or a hydroxyl group, with the proviso that at least one of $R^1$ to $R^6$ represents a substituent represented by following Formula (4b):

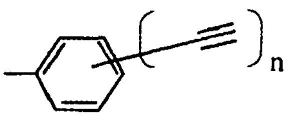

(4b)

wherein "n" denotes a natural number of 1 to 5, wherein when two or more of $R^1$ to $R^6$ represent substituents of Formula (4b), these substituents may be the same as or different from one another, wherein when "n" is 1 in all the substituents (s) represented by Formula (4b) as $R^1$ to $R^6$, then at least one of $R^1$ to $R^6$ is a halo-substituted or unsubstituted alkyl or cycloalkyl group having about six or less carbon atom(s).

According to these processes, ethynylphenylbiadamantane derivatives can be industrially efficiently prepared from halophenylbiadamantane derivatives.

These and other objects, features, and advantages of the present invention will be more fully understood from the following description of preferred embodiments. As used herein, the term "alkyl or cycloalkyl group" refers to an alkyl group, a cycloalkyl group, or a group composed of an alkyl group and a cycloalkyl group bonded to each other.
All numbers are herein assumed to be modified by the term "about."

DESCRIPTION OF THE PREFERRED EMBODIMENTS

Halophenylbiadamantane Derivatives

Halophenylbiadamantane derivatives according to embodiments of the present invention are represented by Formula (1). In Formula (1), $R^1$, $R^2$, $R^3$, $R^4$, $R^5$, and $R^6$ are the same as or different from one another and each represent a hydrogen atom, a halo-substituted or unsubstituted alkyl or cycloalkyl group having about six or less carbon atom(s), a substituted or unsubstituted phenyl group, a carboxyl group, a substituted or unsubstituted carbamoyl group, a substituted oxycarbonyl group, a substituted or unsubstituted amino group, a halogen atom, or a hydroxyl group, with the proviso that at least one of $R^1$ to $R^6$ represents a substituent of Formula (2). In Formula (2), X represents a halogen atom; and "n" denotes a natural number of 1 to 5.

When two or more of $R^1$ to $R^6$ are substituents of Formula (2), these substituents may be the same as or different from one another. The benzene ring in Formula (2) may further have an alkyl or cycloalkyl group having about six or less carbon atoms in addition to the halogen atom(s). When only one of $R^1$ to $R^6$ is a substituent of Formula (2), then at least one of $R^1$ to $R^6$ is a halo-substituted or unsubstituted alkyl or cycloalkyl group having about six or less carbon atom(s) or "n" is a natural number of 2 or more.

As $R^1$ to $R^6$, the halo-substituted or unsubstituted alkyl or cycloalkyl groups each having about six or less carbon atom(s) can be any groups that have an activity of improving the solubility. Exemplary halo-substituted or unsubstituted alkyl groups having about six or less carbon atom(s) include linear or branched alkyl groups having about one to about six carbon atoms, such as methyl, ethyl, propyl, isopropyl, butyl, isobutyl, s-butyl, t-butyl, pentyl, 1,1-dimethylpropyl, 2,2-dimethylpropyl, 3-methylbutyl, hexyl, 4-methylpentyl, 1,3-dimethylbutyl, 1,1-dimethylbutyl, 2-methylpentyl, 3,3-dimethylbutyl, 1,2,2-trimethylpropyl, and 2,2-dimethylbutyl groups; and groups corresponding to these groups, except with at least one halogen atom replacing at least one hydrogen atom thereof.

Exemplary halo-substituted or unsubstituted cycloalkyl groups having about six or less carbon atom(s) as $R^1$ to $R^6$ include cycloalkyl groups having about three to about six members, such as cyclopropyl, cyclobutyl, cyclopentyl, and cyclohexyl groups, of which cycloalkyl groups having five or six members are preferred; and groups corresponding to these groups, except with at least one halogen atom replacing at least one hydrogen atom thereof.

As used herein, the term "alkyl or cycloalkyl group" further includes a group composed of an alkyl group and a cycloalkyl group bonded to each other. Exemplary halo-substituted or unsubstituted groups each containing an alkyl group and a cycloalkyl group bonded to each other having about six or less carbon atom(s) as $R^1$ to $R^6$ include cyclopropylmethyl, cyclopropylethyl, cyclopropylpropyl, 2-methyl-3-ethylcyclopropyl, 2-cyclopropylethyl, 1-ethyl-1-cyclobutyl, 2-cyclobutylethyl, 2,3-dimethylcyclobutyl, 2-ethylcyclobutyl, and cyclopentylmethyl groups; and groups corresponding to these groups, except for having at least one halogen atom replacing at least one hydrogen atom thereof.

The alkyl groups, cycloalkyl groups, and groups each containing an alkyl group and a cycloalkyl group bonded to each other may each have one or more halogen atoms. Exemplary halogen atoms include bromine atom (Br), iodine atom (I), chlorine atom (Cl), and fluorine atom (F).

Exemplary substituents of the substituted or unsubstituted phenyl groups as $R^1$ to $R^6$ include a wide variety of substituents including alkyl groups, cycloalkyl groups, halogen atoms (e.g., fluorine atom, chlorine atom, bromine atom, and iodine atom), hydroxyl group, substituted oxy groups (e.g., alkoxy groups, aryloxy groups, aralkyloxy groups, and acyloxy groups), carboxyl group, substituted oxycarbonyl groups (e.g., alkoxycarbonyl groups, aryloxycarbonyl groups, and aralkyloxycarbonyl groups), substituted or unsubstituted carbamoyl groups, cyano group, nitro group, substituted or unsubstituted amino groups, sulfo group, and heterocyclic groups. The hydroxyl group and carboxyl group as substituents may be protected by any of protecting groups generally used in organic syntheses. The substituted or unsubstituted phenyl group is preferably phenyl group or methylphenyl group.

Exemplary substituted oxycarbonyl groups as $R^1$ to $R^6$ include alkoxycarbonyl groups including alkoxycarbonyl groups whose alkoxy moiety has about one to six carbon atoms, such as methoxycarbonyl and ethoxycarbonyl groups; aryloxycarbonyl groups such as phenoxycarbonyl group; and aralkyloxycarbonyl groups such as benzyloxycarbonyl group.

Exemplary substituted or unsubstituted carbamoyl groups as $R^1$ to $R^6$ include unsubstituted carbamoyl group; and carbamoyl groups having a variety of substituents. Such substituents include alkyl groups such as methyl, ethyl, propyl, isopropyl, n-butyl, s-butyl, and t-butyl groups; and acyl groups such as acetyl and benzoyl groups.

Exemplary substituted or unsubstituted amino groups as $R^1$ to $R^6$ include unsubstituted amino group; and amino groups having a variety of substituents. Such substituents include alkyl groups such as methyl, ethyl, propyl, isopropyl, n-butyl, s-butyl, and t-butyl groups; and acyl groups such as acetyl and benzoyl groups.

The carboxyl group and hydroxyl group as $R^1$ to $R^6$ may each be protected by any of protecting groups generally used in organic syntheses.

In a preferred embodiment, at least one (more preferably two or four) of $R^1$ to $R^6$ is a linear alkyl group or cycloalkyl group having about six or less carbon atom(s). This further improves the solubility in solvents as the monomer (halophenylbiadamantane derivative) and improves the solubility in solvents as a polymer polymerized from the monomer.

In the substituent of Formula (2), X represents a halogen atom; and "n" denotes a natural number of 1 to 5. The benzene ring in Formula (2) may further have an alkyl or cycloalkyl group having about six or less carbon atoms, in addition to the halogen atom(s). Exemplary halogen atoms and exemplary alkyl or cycloalkyl groups each having about six or less carbon atoms(s) are as above.

Exemplary substituents of Formula (2) which have one or more halogen atoms include monobromophenyl groups such as 2-bromophenyl, 3-bromophenyl, and 4-bromophenyl; dibromophenyl groups such as 2,3-dibromophenyl, 2,4-dibromophenyl, 2,5-dibromophenyl, and 3,5-dibromophenyl; tribromophenyl groups such as 2,3,4-tribromophenyl, 3,4,5-tribromophenyl, 2,3,5-tribromophenyl, and 2,3,6-tribromophenyl; and tetrabromophenyl groups such as 2,3,4,5-tetrabromophenyl and 2,3,5,6-tetrabromophenyl.

Exemplary substituents of Formula (2) which have one or more alkyl groups in addition to one or more halogen atoms include monobromophenyl groups such as 3-methyl-2-bromophenyl, 4-methyl-2-bromophenyl, 5-methyl-2-bromophenyl, 6-methyl-2-bromophenyl, 2-methyl-3-bromophenyl, 4-methyl-3-bromophenyl, 5-methyl-3-bromophenyl, 6-methyl-3-bromophenyl, 2-methyl-4-bromophenyl, and 3-methyl-4-bromophenyl; dibromophenyl groups such as 4-methyl-2,3-dibromophenyl, 5-methyl-2,3-dibromophenyl, 6-methyl-2,3-dibromophenyl, 3-methyl-2,4-dibromophenyl, 5-methyl-2,4-dibromophenyl, 6-methyl-2,4-dibromophenyl, 3-methyl-2,5-dibromophenyl, 4-methyl-2,5-dibromophenyl, 6-methyl-2,5-dibromophenyl, 2-methyl-3,5-dibromophenyl, and 4-methyl-3,5-dibromophenyl; tribromophenyl groups such as 5-methyl-2,3,4-tribromophenyl, 6-methyl-2,3,4-tribromophenyl, 2-methyl-3,4,5-tribromophenyl, 4-methyl-2,3,5-tribromophenyl, 6-methyl-2,3,5-tribromophenyl, 4-methyl-2,3,6-tribromophenyl, and 5-methyl-2,3,6-tribromophenyl; and tetrabromophenyl groups such as 6-methyl-2,3,4,5-tetrabromophenyl and 4-methyl-2,3,4,5-tetrabromophenyl.

Exemplary substituents of Formula (2) further include groups corresponding to the above-exemplified groups, except for having iodine atom (I), chlorine atom (Cl), and/or fluorine atom (F) as the halogen atom instead of the bromine atom (Br). In addition, exemplary substituents of Formula (2) further include groups corresponding to the above-exemplified groups, except for having one or more other alkyl or cycloalkyl groups having about six or less carbon atom(s) as mentioned above, instead of the methyl group.

Representative halophenylbiadamantane derivatives of Formula (1), in which all of $R^1$ to $R^6$ are each a hydrogen atom or a substituent of Formula (2), include 3-(3,5-dibromophenyl)-1,1'-biadamantane, 3,3-di(4-bromophenyl)-1,1'-biadamantane, 3,3-di(3,5-dibromophenyl)-1,1'-biadamantane, 3,3',5-tri(4-bromophenyl)-1,1'-biadamantane, 3,3',5-tri (3,5-dibromophenyl)-1,1'-biadamantane, 3,3',5,5'-tetra(4-bromophenyl)-1,1'-biadamantane, and 3,3',5,5'-tetra(3,5-dibromophenyl)-1,1'-biadamantane.

Representative halophenylbiadamantane derivatives of Formula (1), in which all of $R^1$ to $R^6$ are each a hydrogen atom or a substituent of Formula (2), further include compounds corresponding to the above-exemplified compounds, except for having iodine atom (I), chlorine atom (Cl), and/or fluorine atom (F) as the halogen atom instead of the bromine atom (Br).

Representative halophenylbiadamantane derivatives of Formula (1), in which at least one of $R^1$ to $R^6$ is an alkyl or cycloalkyl group having about six or less carbon atom(s) and at least one of $R^1$ to $R^6$ is a substituent of Formula (2), include biadamantane derivatives having methyl group as the alkyl or cycloalkyl group having about six or less carbon atom(s), such as 3-(4-bromophenyl)-5,5',7,7'-tetramethyl-1,1'-biadamantane, 3-(3,5-dibromophenyl)-5,5',7,7'-tetramethyl-1,1'-biadamantane, 3,3'-bis(4-bromophenyl)-5,5',7,7'-tetramethyl-1,1'-biadamantane, and 3,3'-bis(3,5-dibromophenyl)-5,5',7,7'-tetramethyl-1,1'-biadamantane; biadamantane derivatives having ethyl group as the alkyl or cycloalkyl group having about six or less carbon atom(s), such as 3-(4-bromophenyl)-5,5',7,7'-tetraethyl-1,1'-biadamantane, 3-(3,5-dibromophenyl)-5,5',7,7'-tetraethyl-1,1'-biadamantane, 3,3'-bis(4-bromophenyl)-5,5',7,7'-tetraethyl-1,1'-biadamantane, and 3,3'-bis(3,5-dibromophenyl)-5,5',7,7'-tetraethyl-1,1'-biadamantane; and halophenylbiadamantane derivatives having n-butyl group as the alkyl or cycloalkyl group having about six or less carbon atom(s), such as 3-(4-bromophenyl)-5,5',7,7'-tetra(n-butyl)-1,1'-biadamantane, 3-(3,5-dibromophenyl)-5,5',7,7'-tetrakis(n-butyl)-1,1'-biadamantane, 3,3'-bis(4-bromophenyl)-5,5',7,7'-tetrakis(n-butyl)-1,1'-biadamantane, and 3,3'-bis(3,5-dibromophenyl)-5,5', 7,7'-tetrakis(n-butyl)-1,1'-biadamantane.

Exemplary halophenylbiadamantane derivatives of Formula (1), in which at least one of $R^1$ to $R^6$ is an alkyl or cycloalkyl group having about six or less carbon atom(s) and at least one of them is a substituent of Formula (2), further include compounds corresponding to the above-exemplified compounds, except for having iodine atom (I), chlorine atom (Cl), and/or fluorine atom (F) as the halogen atom instead of the bromine atom (Br).

Representative halophenylbiadamantane derivatives of Formula (1), in which all of $R^1$ to $R^6$ are each a hydrogen atom or a substituent of Formula (2) and the benzene ring in Formula (2) has an alkyl or cycloalkyl group having about six or less carbon atom(s) in addition to the halogen atom(s), include 3-(4-methyl-3,5-dibromophenyl)-1,1'-biadamantane, 3-(2-methyl-3,5-dibromophenyl)-1,1'-biadamantane, 3,3'-bis(2-methyl-4-bromophenyl)-1,1'-biadamantane, 3,3'-bis(3-methyl-4-bromophenyl)-1,1'-biadamantane, 3,3'-bis(4-methyl-3,5-dibromophenyl)-1,1'-biadamantane, 3,3'-bis(2-methyl-3,5-dibromophenyl)-1,1'-biadamantane, 3,3',5-tris(2-methyl-4-bromophenyl)-1,1'-biadamantane, 3,3',5-tris(3-methyl-4-bromophenyl)-1,1'-biadamantane, 3,3',5-tris(4-methyl-3,5-dibromophenyl)-1,1'-biadamantane, 3,3',5-tris(2-methyl-3,5-dibromophenyl)-1,1'-biadamantane, 3,3',5,5'-tetrakis(2-methyl-4-bromophenyl)-1,1'-biadamantane, 3,3',5,5'-tetrakis(3-methyl-4-bromophenyl)-1,1-adamantane, 3,3',5,5'-tetrakis(4-methyl-3,5-dibromophenyl)-1,1'-biadamantane, and 3,3',5,5'-tetrakis(2-methyl-3,5-dibromophenyl)-1,1'-biadamantane.

Exemplary halophenylbiadamantane derivatives of Formula (1), in which all of $R^1$ to $R^6$ are each a hydrogen atom or a substituent of Formula (2) and the benzene ring in Formula (2) has an alkyl or cycloalkyl group having about six or less carbon atom(s) in addition to the halogen atom(s), further include compounds corresponding to the above-exemplified compounds, except for having iodine atom (I), chlorine atom (Cl), and/or fluorine atom (F) as the halogen atom instead of the bromine atom (Br). The exemplary halophenylbiadamantane derivatives just mentioned above further include compounds corresponding to the above-exemplified compounds, except for having one or more other alkyl or cycloalkyl groups having about six or less carbon atom(s) as mention above, instead of the methyl group.

Representative halophenylbiadamantane derivatives of Formula (1), in which at least one of $R^1$ to $R^6$ is an alkyl or cycloalkyl group having about six or less carbon atom(s) and at least one of $R^1$ to $R^6$ is a substituent of Formula (2) and the benzene ring in Formula (2) has one or more halogen atoms and one or more alkyl groups, include biadamantane derivatives, in which at least one of $R^1$ to $R^6$ is methyl group as the alkyl or cycloalkyl group having about six or less carbon atom(s), such as 3-(2-methyl-4-bromophenyl)-5,5',7,7'-tetramethyl-1,1'-biadamantane, 3-(3-methyl-4-bromophenyl)-5,5',7,7'-tetramethyl-1,1'-biadamantane, 3-(2-methyl-3,5-dibromophenyl)-5,5',7,7'-tetramethyl-1,1'-biadamantane, 3-(4-methyl-3,5-dibromophenyl)-5,5',7,7'-tetramethyl-1,1'-biadamantane, 3,3'-bis(2-methyl-4-bromophenyl)-5,5',7,7'-tetramethyl-1,1'-biadamantane, 3,3'-bis(3-methyl-4-bromophenyl)-5,5',7,7'-tetramethyl-1,1'-biadamantane, 3,3'-bis(2-methyl-3,5-dibromophenyl)-5,5',7,7'-tetramethyl-1,1'-biadamantane, and 3,3'-bis(4-methyl-3,5-dibromophenyl)-5,5',7,7'-tetramethyl-1,1'-biadamantane; biadamantane derivatives, in which at least one of $R^1$ to $R^6$ is ethyl group as the alkyl or cycloalkyl group having about six or less carbon atom(s), such as 3-(2-methyl-4-bromophenyl)-5,5',7,7'-tetraethyl-1,1'-biadamantane, 3-(3-methyl-4-bromophenyl)-5,5',7,7'-tetraethyl-1,1'-biadamantane, 3-(2-methyl-3,5-dibromophenyl)-5,5',7,7'-tetraethyl-1,1'-biadamantane, 3-(4-methyl-3,5-dibromophenyl)-5,5',7,7'-tetraethyl-1,1'-biadamantane, 3,3'-bis(2-methyl-4-bromophenyl)-5,5',7,7'-tetraethyl-1,1'-biadamantane, 3,3'-bis(3-methyl-4-bromophenyl)-5,5',7,7'-tetraethyl-1,1'-biadamantane, 3,3'-bis(2-methyl-3,5-dibromophenyl)-5,5',7,7'-tetraethyl-1,1'-biadamantane, and 3,3'-bis(4-methyl-3,5-dibromophenyl)-5,5',7,7'-tetraethyl-1,1'-biadamantane; and biadamantane derivatives, in which at least one of $R^1$ to $R^6$ is n-butyl group as the alkyl or cycloalkyl group having about six or less carbon atom(s), such as 3-(2-methyl-4-bromophenyl)-5,5',7,7'-tetrakis(n-butyl)-1,1'-biadamantane, 3-(3-methyl-4-bromophenyl)-5,5',7,7'-tetrakis(n-butyl)-1,1'-biadamantane, 3-(2-methyl-3,5-dibromophenyl)-5,5',7,7'-tetrakis(n-butyl)-1,1'-biadamantane, 3-(2-methyl-3,5-dibromophenyl)-5,5',7,7'-tetrakis(n-butyl)-1,1'-biadamantane, 3,3'-bis(2-methyl-4-bromophenyl)-5,5',7,7'-tetra(n-butyl)-1,1'-biadamantane, 3,3'-bis(2-methyl-4-bromophenyl)-5,5',7,7'-tetrakis(n-butyl)-1,1'-biadamantane, 3,3'-bis(2-methyl-3,5-dibromophenyl)-5,5',7,7'-tetrakis(n-butyl)-1,1'-biadamantane, and 3,3'-bis(4-methyl-3,5-dibromophenyl)-5,5',7,7'-tetrakis(n-butyl)-1,1'-biadamantane.

In addition to these compounds, exemplary halophenylbiadamantane derivatives of Formula (1) further include compounds corresponding to the above-exemplified compounds, except for having iodine atom (I), chlorine atom (Cl), and/or fluorine atom (F) as the halogen atom instead of the bromine atom (Br).

Halophenylbiadamantane derivatives according to embodiments of the present invention may each be prepared from a biadamantane halide derivative or biadamantane alcohol derivative with an aromatic compound or a derivative thereof as starting materials by introducing one or more halogenated benzene rings into the biadamantane skeleton or by introducing one or more benzene rings and subsequently introducing one or more halogen atoms into the biadamantane skeleton. Introduction of one or more benzene rings into the biadamantane skeleton may be conducted typically by a Friedel-Crafts reaction, in which a benzene ring is introduced into a biadamantane skeleton of a biadamantane halide derivative or biadamantane alcohol derivative as a starting material; or by a Grignard reaction, in which a benzene ring is introduced into a biadamantane skeleton of a biadamantane halide derivative as a starting material. It is also acceptable that one or more halogen atoms and/or hydroxyl groups remain in the introduced biadamantane skeleton by adjusting the amount of the aromatic compound or by controlling the reaction conditions such as the temperature and amount of catalyst in the introduction process of a benzene ring.

In the Friedel-Crafts reaction, a benzene ring can be introduced into the biadamantane skeleton in the presence of a Lewis acid catalyst in an electrophilic substitution manner. In the Grignard reaction, a benzene ring can be introduced into the biadamantane ring by a coupling reaction between an organic halide and a Grignard reagent.

Exemplary preparation processes (schemes) of halophenylbiadamantane derivatives according to embodiments of the present invention are shown below. In the schemes, X represents a halogen atom (e.g., fluorine atom, chlorine atom, bromine atom, and iodine atom), $R^1$ to $R^6$ are as defined above. However, halophenylbiadamantane derivatives according to embodiments of the present invention are not limited to those prepared by these processes.

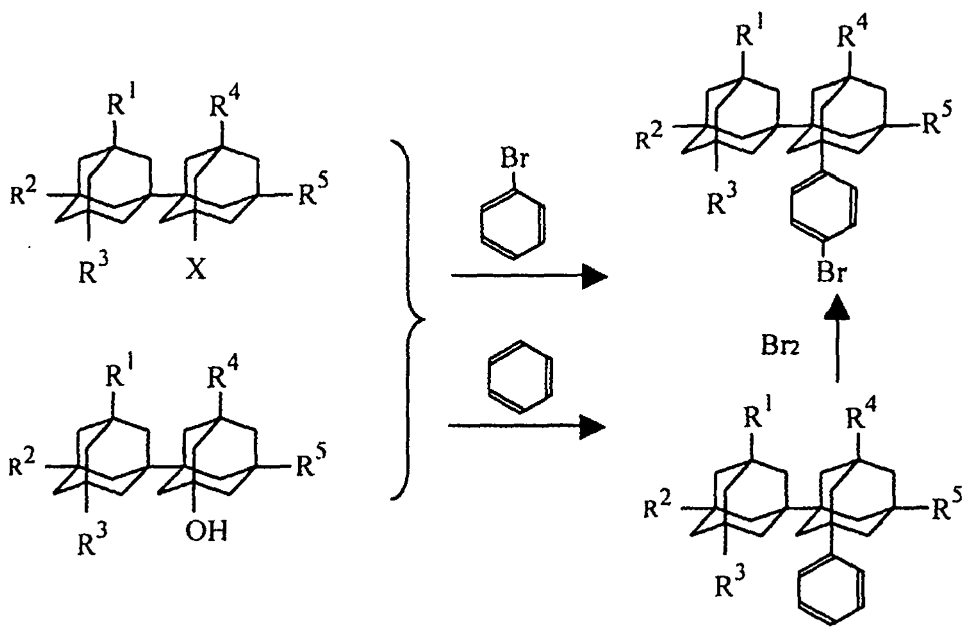

Exemplary biadamantane halide derivatives for use as a starting material include monohalobiadamantane derivatives such as 3-bromo-1,1'-biadamantane, 2-bromo-1,1'-biadamantane, 4-bromo-1,1'-biadamantane, 3-bromo-5,7-dimethyl-1,1'-biadamantane, 3-bromo-5',7'-dimethyl-1,1'-biadamantane, 3-bromo-5,5',7,7'-tetramethyl-1,1'-biadamantane, 3-bromo-5,5',7,7'-tetraethyl-1,1'-biadamantane, 3-bromo-5,5',7,7'-tetrabutyl-1,1'-biadamantane, and 3-bromo-5,5',7,7'-tetrakis(trifluoromethyl)-1,1'-biadamantane; dihalobiadamantane derivatives such as 3,3'-dibromo-1,1'-biadamantane, 2,3'-dibromo-1,1'-biadamantane, 3',4-dibromo-1,1'-biadamantane, 2,2'-dibromo-1,1'-biadamantane, 4,4'-dibromo-1,1'-biadamantane, 3,3'-dibromo-5,7-dimethyl-1,1'-biadamantane, 3,3'-dibromo-5,5',7,7'-tetramethyl-1,1'-biadamantane, 3,3'-dibromo-5,5',7,7'-tetraethyl-1,1'-biadamantane, 3,3'-dibromo-5,5',7,7'-tetrabutyl-1,1'-biadamantane, 3,3'-dibromo-5,5',7,7'-tetrakis(trifluoromethyl)-1,1'-biadamantane, 3,3'-dibromo-5,5',7,7'-tetrahydroxy-1,1'-biadamantane, and 3,3'-dibromo-5,5',7,7'-tetraamino-1,1'-biadamantane; tetrahalobiadamantane derivatives such as 3,3',5,5'-tetrabromo-1,1'-biadamantane; and hexahalobiadamantane derivatives such as 3,3',5,5',7,7'-hexabromo-1,1'-biadamantane.

Exemplary biadamantane alcohol derivatives for use as a starting material include monohydroxybiadamantane derivatives such as 3-hydroxy-1,1'-biadamantane, 2-hydroxy-1,1'-biadamantane, 4-hydroxy-1,1'-biadamantane, 3-hydroxy-5,7-dimethyl-1,1'-biadamantane, 3-hydroxy-5',7'-dimethyl-1,1'-biadamantane, 3-hydroxy-5,5',7,7'-tetramethyl-1,1'-biadamantane, 3-hydroxy-5,5',7,7'-tetraethyl-1,1'-biadamantane, 3-hydroxy-5,5',7,7'-tetrabutyl-1,1'-biadamantane, and 3-hydroxy-5,5',7,7'-tetrakis(trifluoromethyl)-1,1'-biadamantane; dihydroxybiadamantane derivatives such as 3,3'-dihydroxy-1,1'-biadamantane, 2,3'-dihydroxy-1,1'-biadamantane, 3',4-dihydroxy-1,1'-biadamantane, 2,2'-dihydroxy-1,1'-biadamantane, 4,4'-dihydroxy-1,1'-biadamantane, 3,3'-dihydroxy-5,7-dimethyl-1,1'-biadamantane, 3,3'-dihydroxy-5,5',7,7'-tetramethyl-1,1'-biadamantane, 3,3'-dihydroxy-5,5',7,7'-tetraethyl-1,1'-biadamantane, 3,3'-dihydroxy-5,5',7,7'-tetrabutyl-1,1'-biadamantane, 3,3'-dihydroxy-5,5',7,7'-tetrakis(trifluoromethyl)-1,1'-biadamantane, and 3,3'-dihydroxy-5,5',7,7'-tetraamino-1,1'-biadamantane; tetrahydroxybiadamantane derivatives such as 3,3',5,5'-tetrahydroxy-1,1'-biadamantane; and hexahydroxybiadamantane derivatives such as 3,3',5,5',7,7'-hexahydroxy-1,1'-biadamantane.

Exemplary aromatic compounds or derivatives for use as a starting material include bromobenzene, 1,3-dibromobenzene, 1,3,5-tribromobenzene, iodobenzene, 1,3-diiodobenzene, and 1,3,5-triiodobenzene. Among them, bromobenzene and/or 1,3-dibromobenzene is preferably used. The amount of the aromatic compound or a derivative thereof is, for example, about 1 to 1000 moles, preferably about 2 to 500 moles, and more preferably about 5 to 200 moles, to 1 mole of the biadamantane halide derivative or biadamantane alcohol derivative.

The Friedel-Crafts reaction is carried out in the presence of a Lewis acid catalyst. Exemplary Lewis acid catalysts include iron halides such as $FeCl_3$ and $FeBr_3$; aluminum halides such as $AlCl_3$ and $AlBr_3$; zirconium halides such as $ZrCl_2$ and $ZrCl_4$; and $BF_3$. Among them, $AlCl_3$ is advantageously used. The amount of Lewis acid catalysts can be set as appropriate according typically to the reaction rate and is, for example, about 0.001 to 10 moles, and preferably about 0.05 to 5 moles, to 1 mole of the biadamantane halide derivative or biadamantane alcohol derivative.

A Friedel-Crafts reaction, if using a biadamantane alcohol derivative as a starting material for a halophenylbiadamantane derivative, is preferably carried out in the presence of an acid, so as to accelerate the reaction. Exemplary acids include inorganic acids such as sulfuric acid, hydrochloric acid, hydrobromic acid, nitric acid, and phosphoric acid; sulfonic acids such as methanesulfonic acid; carboxylic acids such as acetic acid and propionic acid; heteropolyacids; and cation-exchange resins. Among them, preferred are strong acids including inorganic acids such as hydrochloric acid and sulfuric acid; sulfonic acids such as p-toluenesulfonic acid; heteropolyacids; and strongly acidic cation-exchange resins. The amount of acids is, for example, about 0.1 to 10 moles, and preferably about 0.1 to 5 moles, to 1 mole of the biadamantane alcohol derivative.

The Friedel-Crafts reaction is carried out in the presence of, or in the absence of, an inert solvent. Exemplary solvents include hydrocarbons such as hexane and cyclohexane; halogenated hydrocarbons such as methylene chloride, 1,2-dichloroethane, chloroform, and carbon tetrachloride; chain or cyclic ethers such as diethyl ether, dimethoxyethane, tetrahydrofuran, and dioxane; nitriles such as acetonitrile and benzonitrile; esters such as ethyl acetate; carboxylic acids such as acetic acid; amides such as N,N-dimethylformamide; ketones such as acetone and methyl ethyl ketone; nitro compounds such as nitromethane and nitrobenzene; and mixtures of these solvents.

The reaction temperature may be suitably set according typically to the types of reaction components. Typically, when a biadamantane halide derivative is used, the reaction temperature is, for example, about -50°C to 200°C, preferably about -20°C to 150°C. When a biadamantane alcohol derivative is used, the reaction temperature is, for example, about 10°C to 200°C, preferably about 40°C to 150°C, and more preferably about 60°C to 130°C. The reaction may be conducted under normal atmospheric pressure, under reduced pressure, or under a pressure (under a load). The reaction atmosphere is not particularly limited, as long as it does not adversely affect the reaction, and can be any of, for example, air atmosphere, nitrogen atmosphere, and argon atmosphere. The reaction may be conducted according to any system such as a batch system, semi-batch system, or continuous system.

In the Grignard reaction, the Grignard reagent is synthetically prepared by a reaction between magnesium and a halogen-containing compound. Exemplary Grignard reagents for use herein include phenylmagnesium bromide, phenylmagnesium iodide, and phenylmagnesium chloride, of which phenylmagnesium bromide is preferred. These Grignard

reagents may be those formed in a reactor in situ or in another vessel or may be commercially available products. The amount of the Grignard reagents is, for example, about 1 to 2 moles, preferably about 1 to 1.5 moles, and more preferably about 1 to 1.2 moles, to 1 mole of the biadamantane halide derivative.

The Grignard reaction is carried out in a solvent. It is preferred that the Grignard reaction is conducted in an anhydrous or absolute ether solvent in an inert atmosphere, because this reaction uses a Grignard reagent. Preferred exemplary ether solvents are diethyl ether, diisopropyl ether, and tetrahydrofuran, of which tetrahydrofuran is more preferred.

The reaction temperature may be suitably set according typically to the types of reaction components and catalysts and is, for example, about -20°C to 80°C, and preferably about -10°C to 50°C. The reaction may be conducted under normal atmospheric pressure, under reduced pressure, or under a pressure (under a load). The reaction atmosphere is not particularly limited, as long as it does not adversely affect the reaction, and can be any of, for example, air atmosphere, nitrogen atmosphere, and argon atmosphere. The reaction may be conducted according to any system such as a batch system, semi-batch system, or continuous system.

When a reaction product is obtained in a high yield after the reaction but the reaction mixture contains an acid catalyst, the purification of the reaction product may be conducted by a general purification procedure for removing catalysts, such as adjustment of pH of the reaction mixture, concentration, filtration, and/or washing. When the reaction mixture contains large amounts of by-products, the purification can be conducted by a common separation and purification procedure such as crystallization, recrystallization, or column chromatography, or any combination of these procedures.

The crystallization is carried out by dissolving the reaction mixture in a suitable solvent where necessary with heating, concentrating the solution according to necessity, and cooling the solution to precipitate crystals. Exemplary solvents include ethers such as tetrahydrofuran; ketones such as acetone; hydrocarbons such as toluene; halogenated hydrocarbons such as chloroform; and mixtures of these solvents. The crystallization is conducted, for example, by using a combination of a good solvent (e.g., tetrahydrofuran or toluene) and a poor solvent (e.g., methanol) or by using a combination of water and a water-miscible organic solvent (e.g., tetrahydrofuran, 1,4-dioxane, acetone, or acetonitrile).

The halophenylbiadamantane derivatives according to embodiments of the present invention excel in solubility in solvents, and whose halogen atom can be converted into a substituent of various kind, such as carbonyl group, alkyl group, phenyl group, alkenyl group, or alkynyl group (e.g., ethynyl group), and are widely usable as starting materials for a variety of adamantane derivatives.

Ethynylphenylbiadamantane Derivatives

Ethynylphenylbiadamantane derivatives according to embodiments of the present invention are represented by Formula (3). In Formula (3), $R^1$, $R^2$, $R^3$, $R^4$, $R^5$, and $R^6$ are the same as or different from one another and each represent a hydrogen atom, a halo-substituted or unsubstituted alkyl or cycloalkyl group having about six or less carbon atom(s), a substituted or unsubstituted phenyl group, a carboxyl group, a substituted or unsubstituted carbamoyl group, a substituted oxycarbonyl group, a substituted or unsubstituted amino group, a halogen atom, or a hydroxyl group, with the proviso that at least one of $R^1$ to $R^6$ represents a substituent of Formula (4). In Formula (4), Y represents a hydrogen atom, an alkyl group having about one to about six carbon atoms, a phenyl group, or a tri-substituted silyl group; and "n" denotes a natural number of 1 to 5.

When two or more of $R^1$ to $R^6$ represent substituents of Formula (4), these substituents may be the same as or different from one another. When "n" is 1 in all the substituent(s) of Formula (4) as $R^1$ to $R^6$, then at least one of $R^1$ to $R^6$ is a halo-substituted or unsubstituted alkyl or cycloalkyl group having about six or less carbon atom(s).

In Formula (3), exemplary groups as $R^1$ to $R^6$ are as with $R^1$ to $R^6$ in Formula (1) . In Formula (4), exemplary alkyl groups having about one to about six carbon atoms as Y include linear or branched alkyl groups having about one to about six carbon atoms, such as methyl, ethyl, propyl, isopropyl, butyl, isobutyl, s-butyl, t-butyl, pentyl, 1,1-dimethylpropyl, 2,2-dimethylpropyl, 3-methylbutyl, hexyl, 4-methylpentyl, 1,3-dimethylbutyl, 1,1-dimethylbutyl, 2-methylpentyl, 3,3-dimethylbutyl, 1,2,2-trimethylpropyl, and 2,2-dimethylbutyl groups. When Y is a tri-substituted silyl group, the three substituents may be any of alkyl groups, cycloalkyl groups, groups containing an alkyl group and a cycloalkyl group bonded to each other, and phenyl group, and they may be the same as or different from one another.

In the tri-substituted silyl group as Y, exemplary alkyl groups as the substituents of silyl group include linear or branched alkyl groups having about one to about six carbon atoms, such as methyl, ethyl, propyl, isopropyl, butyl, isobutyl, s-butyl, t-butyl, pentyl, 1,1-dimethylpropyl, 2,2-dimethylpropyl, 3-methylbutyl, hexyl, 4-methylpentyl, 1,3-dimethylbutyl, 1,1-dimethylbutyl, 2-methylpentyl, 3,3-dimethylbutyl, 1,2,2-trimethylpropyl, and 2,2-dimethylbutyl groups.

In the tri-substituted silyl group as Y, exemplary cycloalkyl groups as the substituents of silyl group include cycloalkyl groups having about three to about six members, such as cyclopropyl, cyclobutyl, cyclopentyl, and cyclohexyl groups, of which cycloalkyl groups having about five or six members are preferred.

In the tri-substituted silyl group as Y, exemplary groups containing an alkyl group and a cycloalkyl group bonded to each other as the substituents of silyl group include groups containing an alkyl group and a cycloalkyl group having

about six or less carbon atom(s), such as cyclopropylmethyl, cyclopropylethyl, cyclopropylpropyl, 2-methyl-3-ethylcyclopropyl, 2-cyclopropylethyl, 1-ethyl-1-cyclobutyl, 2-cyclobutylethyl, 2,3-dimethylcyclobutyl, 2-ethylcyclobutyl, and cyclopentylmethyl groups.

Exemplary ethynylphenylbiadamantane derivatives, in which all of $R^1$ to $R^6$ are independently a hydrogen atom or a group of Formula (4) and Y is a hydrogen atom, include 3-(3,5-diethynylphenyl)-1,1'-biadamantane; 3,3'-bis(3,5-diethynylphenyl)-1,1'-biadamantane; and 3,3',5,5'-tetrakis(3,5-diethynylphenyl)-1,1'-biadamantane.

Exemplary ethynylphenylbiadamantane derivatives, in which all of $R^1$ to $R^6$ are independently a hydrogen atom or a group of Formula (4) and Y is a trimethylsilyl group, include 3-(3,5-bis(trimethylsilylethynyl)phenyl)-1,1'-biadamantane; 3,3'-bis(3,5-bis(trimethylsilylethynyl)phenyl)-1,1'-biadamantane; and 3,3',5,5'-tetrakis(3,5-bis(trimethylsilylethynyl) phenyl)-1,1'-biadamantane.

Exemplary ethynylphenylbiadamantane derivatives, in which all of $R^1$ to $R^6$ are independently a hydrogen atom or a group of Formula (4) and Y is a tri-substituted silyl group, include compounds corresponding to the above-exemplified ethynylphenylbiadamantane derivatives, except with any of the above-exemplified substituents of silyl group instead of the methyl group.

Exemplary ethynylphenylbiadamantane derivatives, in which all of $R^1$ to $R^6$ are independently a hydrogen atom or a group of Formula (4) and Y is a phenyl group, include 3-(3,5-bis(phenylethynyl)phenyl)-1,1'-biadamantane; 3, 3'-bis(3,5-bis(phenylethynyl)phenyl)-1,1'-biadamantane; and 3,3',5,5'-tetrakis(3,5-bis(phenylethynyl)phenyl)-1,1'-biadamantane.

Exemplary ethynylphenylbiadamantane derivatives, in which at least one of $R^1$ to $R^6$ is an alkyl or cycloalkyl group having about six or less carbon atom(s) and at least one of them is a substituent of Formula (4), and Y is a hydrogen atom, include 3-(4-ethynylphenyl)-5,5',7,7'-tetramethyl-1,1'-biadamantane, 3, 3'-bis(4-ethynylphenyl)-5,5',7,7'-tetramethyl-1,1'-biadamantane; 3-(3,5-diethynylphenyl)-5,5',7,7'-tetramethyl-1,1'-biadamantane; and 3,3'-bis(3,5-diethynylphenyl)-5,5',7,7'-tetramethyl-1,1'-biadamantane.

Exemplary ethynylphenylbiadamantane derivatives, in which at least one of $R^1$ to $R^6$ is an alkyl or cycloalkyl group having about six or less carbon atom(s) and at least one of them is a substituent of Formula (4), and Y is a trimethylsilyl group, include 3-(4-(trimethylsilylethynyl)phenyl)-5,5',7,7'-tetramethyl-1,1'-biadamantane, 3,3'-bis(4-(trimethylsilylethynyl)phenyl)-5,5',7,7'-tetramethyl-1,1'-biadamantane, ; 3-(3,5-bis(trimethylsilylethynyl)phenyl)-5,5',7,7'-tetramethyl-1,1'-biadamantane; and 3,3'-bis(3,5-bis(trimethylsilylethynyl)phenyl)-5,5',7,7'-tetramethyl-1,1'-biadamantane.

Exemplary ethynylphenylbiadamantane derivatives, in which at least one of $R^1$ to $R^6$ is an alkyl or cycloalkyl group having about six or less carbon atom(s) and at least one of them is a substituent of Formula (4), and Y is a phenyl group, include 3-(4-(phenylethynyl)phenyl)-5,5',7,7'-tetramethyl-1,1'-biadamantane, 3,3'-bis(4-(phenylethynyl)phenyl)-5,5',7,7'-tetramethyl-1,1'-biadamantane; 3-(3,5-bis(phenylethynyl)phenyl)-5,5',7,7'-tetramethyl-1,1'-biadamantane; and 3,3'-bis(3,5-bis(phenylethynyl)phenyl)-5,5',7,7'-tetramethyl-1,1'-biadamantane.

Exemplary ethynylphenylbiadamantane derivatives, in which at least one of $R^1$ to $R^6$ is an alkyl or cycloalkyl group having about six or less carbon atom(s) and at least one of them is a substituent of Formula (4) further include compounds corresponding to the above-exemplified ethynylphenylbiadamantane derivatives, except for having one or more other alkyl or cycloalkyl groups having about six or less carbon atom(s) as mentioned above, instead of the methyl group.

Process (1) for the Preparation of Ethynylphenylbiadamantane Derivatives

A process according to an embodiment of the present invention is a process for the preparation of an ethynylphenylbiadamantane derivative of Formula (3) from a halophenylbiadamantane derivative of Formula (1a) and a terminal alkyne of Formula (5) as starting materials through a cross coupling reaction (Sonogashira reaction). Such ethynylphenylbiadamantane derivatives of Formula (3) can also be prepared through a coupling reaction between a halophenylbiadamantane derivative of Formula (1a) and a copper acetylide (Castro reaction).

In the cross coupling reaction (Sonogashira reaction), a halophenylbiadamantane derivative of Formula (1a) and a terminal alkyne of Formula (5) are reacted through a cross coupling reaction in the presence typically of a palladium catalyst, a copper salt as a promoter, a ligand, and/or a basic compound, to give an ethynylphenylbiadamantane derivative of Formula (3). The copper salt and ligand may be used or not used in the reaction. In a preferred embodiment, they are used in the reaction, to proceed the reaction under milder conditions.

In Formula (5), R' represents a hydrogen atom or a metal atom such as copper, zinc, or tin. Y in Formula (5) corresponds to Y in the ethynylphenylbiadamantane derivative of Formula (3) and examples of the former Y are as with the latter Y. In a preferred embodiment, the terminal alkyne of Formula (5) is a mono-substituted acetylene. In a more preferred embodiment, the mono-substituted acetylene is trimethylsilylacetylene, phenylacetylene, and/or adamantylacetylene. Exemplary terminal alkynes further include an alkynyl copper, an alkynyl zinc, and an alkynyl tin.

The amount of the terminal alkyne of Formula (5) is, for example, about 1.0 to 2.0 moles, preferably about 1.0

to 1.5 moles, and more preferably about 1.0 to 1.2 moles, to 1 mole of halogen atoms in the halophenylbiadamantane derivative of Formula (1a).

Exemplary palladium catalysts for use herein include inorganic compounds including elementary palladium (metal), palladium oxides, palladium sulfides, palladium hydroxides, palladium halides (fluoride, chloride, bromide, and iodide), palladium sulfates, and inorganic palladium complexes; and organic compounds such as palladium cyanides, palladium salts of organic acids (e.g., acetates), and organic palladium complexes. Among them, preferred are palladium halides such as palladium chloride, palladium bromide, and palladium iodide; and palladium complexes.

Exemplary ligands of complexes include known lignans such as triphenylphosphine, trialkylphosphine, tricyanophenylphosphine, tri-O-toluylphosphine, and acetic acid, of which triphenylphosphine is preferred.

Exemplary palladium complexes include dichlorobis(triphenylphosphine)palladium, dibromobis(triphenylphosphine)palladium, diiodobis(triphenylphosphine)palladium, dichlorobis(trimethylphosphine)palladium, dibromobis(trimethylphosphine)palladium, diiodobis(trimethylphosphine)palladium, dichlorobis(tricyanophenylphosphine)palladium, dibromobis(tricyanophenylphosphine)palladium, diiodobis(tricyanophenylphosphine)palladium, dichlorotetrakis(triphenylphosphine)palladium, dibromotetrakis(triphenylphosphine)palladium, and diiodotetrakis(triphenylphosphine)palladium. Each of these palladium catalysts can be used alone or in combination.

The palladium catalysts can be used as intact or as supported by a carrier (support). Exemplary carriers include those commonly used for supporting catalysts, including inorganic metal oxides such as silica, alumina, silica-alumina, zeolite, titania, and magnesia; and activated carbons. The amount of palladium catalysts, if supported by a carrier, is for example, about 0.1 to 50 percent by weight, and preferably about 1 to 20 percent by weight, to the amount of the carrier. Supporting of the catalysts may be conducted according to a common procedure such as impregnation, precipitation, or ion-exchanging.

The amount of palladium catalysts is, for example, about 0.0001 to 10 moles, preferably about 0.001 to 1 mole, and more preferably about 0.002 to 0.5 moles, to 1 mole of halogen atoms in the halophenylbiadamantane derivative of Formula (1a) used as a reaction component.

In a preferred embodiment, triphenylphosphine and/or tricyanophenylphosphine are used as the ligand. The amount of the ligand is, for example, about 0.0001 to 50 moles, preferably about 0.001 to 20 moles, and more preferably about 0.002 to 10 moles, to 1 mole of halogen atoms in the halophenylbiadamantane derivative of Formula (1a).

Exemplary basic compounds include alkylamines such as trimethylamine, triethylamine, diethylamine, diisopropylamine, n-butylamine, ethylenediamine, and dimetylaminopropionitrile (DMAP); aromatic amine compounds such as aniline; heteroaromatic amine compounds such as pyridine, and picoline; cyclic amines such as diazabicycloundecene and piperidine; aminoalcohols such as ethanolamine and diethanolamine; and inorganic bases such as sodium bicarbonate and potassium carbonate. Each of these basic compounds can be used alone or in combination. The amount of basic compounds is, for example, about 1 to 1000 moles, and preferably about 1 to 500 moles, to 1 mole of halogen atoms in the halophenylbiadamantane derivative of Formula (1a).

Exemplary copper salts include copper halides such as copper fluoride, copper chloride, copper bromide, and copper iodide. Each of these copper salts can be used alone or in combination. The amount of copper salts is, for example, about 0.0001 to 10 moles, preferably about 0.001 to 1 mole, and more preferably about 0.002 to 0.5 moles, to 1 mole of halogen atoms in the halophenylbiadamantane derivative of Formula (1a).

In the Sonogashira reaction, the reaction is carried out in the presence of, or in the absence of, a solvent. Exemplary solvents include hydrocarbons such as hexane, heptane, and cyclohexane; aromatic hydrocarbons such as benzene, toluene, xylenes, and mesitylene; halogenated hydrocarbons such as methylene chloride, 1,2-dichloroethane, chloroform, and carbon tetrachloride; chain or cyclic ethers such as diethyl ether, dimethoxyethane, tetrahydrofuran, dioxolane, and diethylene glycol dimethyl ether (diglyme); nitriles such as acetonitrile and benzonitrile; esters such as ethyl acetate and butyl acetate; amides such as N,N-dimethylformamide, N,N-dimethylacetamide, and N-methyl-2-pyrrolidone; ketones such as acetone, methyl ethyl ketone, and cyclohexanone; and nitro compounds such as nitromethane and nitrobenzene. Each of these solvents can be used alone or in combination. Among them, toluene, xylenes, tetrahydrofuran, N,N-dimethylformamide, and N,N-dimethylacetamide are preferred for better solubility. The amount of solvents is, for example, about 0.1 to 1000 parts by weight, and preferably about 1 to 100 parts by weight, to 1 part by weight of the halophenylbiadamantane derivative of Formula (1a).

The reaction temperature may be suitably set according typically to the type of the halophenylbiadamantane derivative used as a starting material, and the reaction rate, and is generally about -20°C to 200°C, preferably about -10°C to 150°C, and more preferably about 0°C to 130°C.

The reaction may be conducted under normal atmospheric pressure, under reduced pressure, or under a pressure (under a load). The reaction atmosphere is not particularly limited, as long as it does not adversely affect the reaction, and can be any of, for example, air atmosphere, nitrogen atmosphere, and argon atmosphere. The reaction may be conducted according to any system such as a batch system, semi-batch system, or continuous system. In a preferred embodiment, the reaction is conducted by mixing the halophenylbiadamantane derivative as a starting material and other components such as a palladium catalyst, a copper salt, a ligand, and a basic compound with a solvent to

give a mixture, and adding the terminal alkyne dropwise to the mixture.

The reaction product may be separated and purified by a procedure such as concentration, crystallization, filtration, washing, recrystallization, precipitation, reprecipitation, extraction, or column chromatography, or any combination of these procedures. Typically, the catalyst, salt, and other unnecessary components may be separated after the completion of reaction by filtration or by adding water and an organic solvent separable from water (e.g., toluene, xylenes, ethyl acetate, or heptane) to the reaction mixture and conducting solution washing. When the reaction mixture contains large amounts of by-products, the reaction product may be separated and purified using a common separation and purification procedure such as crystallization, recrystallization, and/or column chromatography.

The crystallization is carried out by dissolving the reaction mixture in a suitable solvent where necessary with heating, concentrating the solution according to necessity, and cooling the solution to precipitate crystals. Exemplary solvents include ethers such as tetrahydrofuran; ketones such as acetone; hydrocarbons such as toluene; halogenated hydrocarbons such as chloroform; alcohols such as ethanol; and mixtures of these solvents. The crystallization is conducted, for example, by using a combination of a good solvent (e.g., toluene) and a poor solvent (e.g., acetone or methanol), or by using a combination of water and a water-miscible organic solvent (e.g., tetrahydrofuran, 1,4-dioxane, acetone, methanol, ethanol, butanol, or acetonitrile).

In the Castro reaction, a copper acetylide and a halophenylbiadamantane derivative of Formula (1a) are reacted with heating in the presence of pyridine or N,N-dimethylformamide, to give a corresponding ethynylphenylbiadamantane derivative of Formula (3).

The copper acetylide may have, as substituents on its ethynyl group, copper atom and any of the substituent as mentioned above as the substituents in Y. Exemplary copper acetylides include trimethylsilylacetylene copper and phenylacetylene copper.

The amount of copper acetylides is, for example, about 1 to 10 moles, and preferably about 1.2 to 5 moles, to 1 mole of the halophenylbiadamantane derivative of Formula (1a). The reaction temperature is typically about 0°C to 200°C, and preferably about 50°C to 150°C.

The reaction may be conducted under normal atmospheric pressure, under reduced pressure, or under a pressure (under a load). The reaction atmosphere is not particularly limited, as long as it does not adversely affect the reaction, and can be any of, for example, air atmosphere, nitrogen atmosphere, and argon atmosphere. The reaction may be conducted according to any system such as a batch system, semi-batch system, or continuous system.

The reaction product may be separated and purified by a procedure such as concentration, crystallization, filtration, washing, recrystallization, precipitation, reprecipitation, extraction, or column chromatography, or any combination of these procedures. Typically, the catalyst, salt, and other unnecessary components may be separated after the completion of reaction by filtration or by adding water and an organic solvent separable from water (e.g., toluene, xylene, ethyl acetate, or heptane) to the reaction mixture and conducing solution washing. When the reaction mixture contains large amounts of by-products, the reaction product may be separated and purified using a common separation and purification procedure such as crystallization, recrystallization, and/or column chromatography.

The crystallization is carried out by dissolving the reaction mixture in a suitable solvent where necessary with heating, concentrating the solution according to necessity, and cooling the solution to precipitate crystals. Exemplary solvents include ethers such as tetrahydrofuran; ketones such as acetone; hydrocarbons such as toluene; halogenated hydrocarbons such as chloroform; alcohols such as ethanol; and mixtures of these. The crystallization is conducted, for example, by using a combination of a good solvent (e.g., toluene) and a poor solvent (e.g., acetone or methanol), by using a combination of water and a water-miscible organic solvent (e.g., tetrahydrofuran, 1,4-dioxane, acetone, methanol, ethanol, butanol, or acetonitrile).

Process (2) for the Preparation of Ethynylphenylbiadamantane Derivatives

An ethynylphenylbiadamantane derivative of Formula (3b) is prepared by reacting an ethynylphenylbiadamantane derivative of Formula (3a) having a protecting silyl group with an alcohol in the presence of a base to deprotect (remove) the protecting silyl group.

Exemplary bases include common inorganic bases including carbonates of alkali metals, such as potassium carbonate and sodium carbonate; hydroxides of alkali metals, such as sodium hydroxide and potassium hydroxide; and hydrogen carbonates of alkali metals, such as sodium hydrogen carbonate. The amount of bases is, for example, about 0.0001 to 10 moles, preferably about 0.001 to 1 mole, and more preferably about 0.001 to 0.5 moles, to 1 mole of silicon atoms in the ethynylphenylbiadamantane derivative of Formula (3a).

Exemplary alcohols to be reacted with the ethynylphenylbiadamantane derivative of Formula (3a) include primary alcohols, secondary alcohols, and tertiary alcohols. Each of these alcohols may one or more hydroxyl groups and can be any of monohydric alcohols, dihydric alcohols, and polyhydric alcohols.

Representative primary alcohols include saturated or unsaturated aliphatic primary alcohols having about one to thirty carbon atoms, such as methanol, ethanol, 1-propanol, 1-butanol, 2-methyl-1-propanol, 1-hexanol, 1-octanol, 1-decanol, 1-hexadecanol, 2-buten-1-ol, ethylene glycol, trimethylene glycol, glycerol, hexamethylene glycol, and pentaerythritol, of which those having about one to twenty carbon atoms are preferred, those having about one to fifteen carbon atoms are more preferred, and those having about one to about six carbon atoms are further preferred; and primary alcohols having an alicyclic hydrocarbon group (e.g., a cycloalkyl group) bonded to the carbon atom to which a hydroxyl group is bonded, such as cyclopropylmethyl alcohol and 2-cyclopropylethyl alcohol.

Exemplary secondary alcohols include saturated or unsaturated aliphatic secondary alcohols having about three to thirty carbon atoms, such as 2-propanol, s-butyl alcohol, 2-pentanol, 3-pentanol, 3,3-dimethyl-2-butanol, 2-octanol, 4-decanol, 2-hexadecanol, 2-penten-4-ol, and vicinal diols such as glycerol, 1,2-propanediol, 2,3-butanediol, and 2,3-pentanediol, of which those having about three to twenty carbon atoms are preferred, those having about three to fifteen carbon atoms are more preferred, and those having about three to six carbon atoms are further preferred; secondary alcohols having an aliphatic hydrocarbon group and an alicyclic hydrocarbon group (e.g., a cycloalkyl group) bonded to the carbon atom to which a hydroxyl group is bonded, such as 1-cyclopentylethanol and 1-cyclohexylethanol; and saturated or unsaturated alicyclic secondary alcohols (inclusive of bridged secondary alcohols) having about three to twenty members, such as cyclobutanol, cyclopentanol, and cyclohexanol, of which those having about three to fifteen members are preferred, and those having about three to six members are more preferred.

Exemplary tertiary alcohols include saturated or unsaturated aliphatic tertiary alcohols having about four to thirty carbon atoms, such as t-butyl alcohol and t-amyl alcohol, of which those having about four to twenty carbon atoms are preferred, those having about four to fifteen carbon atoms are more preferred, and those having about four to six carbon atoms are further preferred; tertiary alcohols having an aliphatic hydrocarbon group and an alicyclic hydrocarbon group (e.g., a cycloalkyl group or a bridged hydrocarbon group) bonded to the carbon atom to which hydroxyl group is bonded, such as 1-cyclohexyl-1-methylethanol; and tertiary alcohols having a hydroxyl group and an aliphatic hydrocarbon group bonded to one carbon atom constituting an alicyclic ring (e.g., cycloalkane ring or a bridged carbon ring), such as 1-methyl-1-cyclohexanol.

Among these alcohols, methanol, ethanol, propanol, 2-propanol, and butanol are preferably used. The amount of alcohols is, for example, about 0.1 to 1000 moles, and preferably about 1 to 100 moles, to 1 mole of silicon atoms in the ethynylphenylbiadamantane derivative of Formula (3a).

The deprotecting reaction of silyl group is carried out in the presence of, or in the absence of, an inert solvent. Exemplary solvents include water; hydrocarbons such as hexane, heptane, and cyclohexane; aromatic hydrocarbons such as benzene, toluene, xylenes, and mesitylene; halogenated hydrocarbons such as methylene chloride, 1,2-dichloroethane, chloroform, and carbon tetrachloride; chain or cyclic ethers such as diethyl ether, dimethoxyethane, tetrahydrofuran, dioxolane, and diglyme; nitriles such as acetonitrile and benzonitrile; esters such as ethyl acetate and butyl acetate; amides such as N,N-dimethylformamide, N,N-dimethylacetamide, and N-methyl-2-pyrrolidone; ketones such as acetone, methyl ethyl ketone, and cyclohexanone; and nitro compounds such as nitromethane and nitrobenzene. Each of these solvents can be used alone or in combination.

The reaction temperature is, for example, about -20°C to 200°C, preferably about -10°C to 150°C, and more preferably about 0°C to 100°C.

The reaction may be conducted under normal atmospheric pressure, under reduced pressure, or under a pressure (under a load). The reaction atmosphere is not particularly limited, as long as it does not adversely affect the reaction, and can be any of, for example, air atmosphere, nitrogen atmosphere, and argon atmosphere. The reaction may be conducted according to any system such as a batch system, semi-batch system, or continuous system.

The reaction product may be separated and purified by a procedure such as concentration, crystallization, filtration, washing, recrystallization, precipitation, reprecipitation, extraction, or column chromatography, or any combination of these procedures. Typically, the catalyst, salt, and other unnecessary components may be separated after the completion of reaction by filtration or by adding water and an organic solvent separable from water (e.g., toluene or ethyl acetate) to the reaction mixture and conducing solution washing. When the reaction mixture contains large amounts of by-products, the reaction product may be separated and purified using a common separation and purification procedure such as crystallization, recrystallization, and/or column chromatography.

The crystallization is carried out by dissolving the reaction mixture in a suitable solvent where necessary with heating, concentrating the solution according to necessity, and cooling the solution to precipitate crystals. Exemplary solvents include ethers such as tetrahydrofuran; ketones such as acetone; hydrocarbons such as toluene; halogenated hydrocarbons such as chloroform; alcohols such as ethanol; and mixtures of these solvents. The crystallization is conducted, for example, by using a combination of a good solvent (e.g., tetrahydrofuran) and a poor solvent (e.g., methanol) or by using a combination of water and a water-miscible organic solvent (e.g., tetrahydrofuran, 1,4-dioxane, acetone, methanol, ethanol, butanol, or acetonitrile).

Ethynylphenylbiadamantane derivatives according to embodiments of the present invention excel in solubility in solvents and have satisfactory properties such as thermal stability, water resistance, optical properties, optical transparency, low dielectric constant, and stiffness, and are thereby usable as starting materials to give functional materials that excel typically in electrical properties, thermal properties, mechanical properties, optical properties, and/or physical

properties.

Examples

The present invention will be illustrated in further detail with reference to several examples below. It should be noted, however, these are illustrated only by way of example and are never construed to limit the scope of the present invention.

Example 1

In a 1-liter four-necked flask in a nitrogen atmosphere were placed 11 g (0.04 mol) of aluminum bromide and 852 g (3.61 mol) of 1,3-dibromobenzene, and the mixture was cooled with stirring to 5°C. The mixture was combined with 50 g (0.1 mol) of 3,3'-dibromo-5,5',7,7'-tetramethyl-1,1'-biadamantane, followed by stirring at 5°C for 3 hours and sequentially stirring at 20°C for 8 hours. After the completion of reaction, 50 g of 5 N hydrochloric acid and 200 g of water were gradually added to the reaction mixture while cooling to 10°C. The mixture was further combined with 250 g of chloroform, followed by separation. The lower layer was washed with two portions of 300 g of water, separated, and the precipitates were removed by filtration. The filtrate was combined with 700 g of toluene, heated to 50°C, and stirred for 30 minutes. The mixture was cooled to room temperature to give precipitates, and the precipitates were collected by filtration, washed with toluene and methanol, dried at 80°C under reduced pressure, and thereby yielded 73.8 g (in a yield of 90%) of 3,3'-bis(3,5-dibromophenyl)-5,5',7,7'-tetramethyl-1,1'-biadamantane represented by following Formula (6):

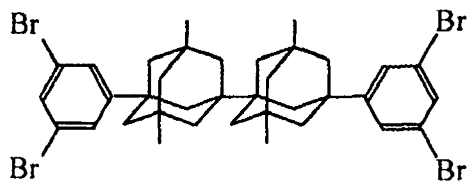

(6)

$^1$H-NMR ($CDCl_3$, 500 MHz) δ: 0.85 (s, 12H), 1.05 (s, 4H), 1.17 (s, 8H), 1.29-1.46 (d, 12H), 7.33 (s, 4H), 7.41 (s, 4H) DI/MS-spectrometry: 794, 559, 397 m/z [electron ionization (EI) detection]

Example 2

In a 1-liter four-necked flask in a nitrogen atmosphere were placed 0.34 g of iron(III) chloride, 80 g of benzene, and 10 g of 3,3'-dibromo-5,5',7,7'-tetramethyl-1,1'-biadamantane, and the mixture was heated to 70°C, followed by stirring for 3 hours. The reaction mixture after the reaction was cooled to 40°C, combined with 30 g of water, stirred for 1 hour, and separated. The upper layer was concentrated and combined with 20 g of acetone, followed by stirring at 20°C for 1 hour. The precipitated crystals were collected by filtration, and washed with acetone. Drying at 50°C under reduced pressure gave 14.2 g (in a yield of 97%) of 3,3'-di(phenyl)-5,5',7,7'-tetramethyl-1,1'-biadamantane. $^1$H-NMR ($CDCl_3$, 500 MHz) δ: 0.83 (s, 12H), 1.02 (q, 4H), 1.19 (q, 8H), 1.34-1.47 (m, 12H), 7.11 (t, 2H), 7.24 (t, 4H), 7.29 (d, 4H) GC/MS spectrometry: 478 m/z [electron ionization (EI) detection]

In a 1-liter four-necked flask in a nitrogen atmosphere were placed 9 g of the above-prepared 3,3'-di(phenyl)-5,5',7,7'-tetramethyl-1,1'-biadamantane, 0.6 g of pyridine, 63 g of chloroform, and 7.4 g of bromine, and the mixture was stirred at 30°C to 40°C for 18 hours. The reaction mixture after the reaction was cooled to 5°C, and combined with an aqueous sodium sulfite solution to remove excess of bromine. The mixture was separated, and precipitates in the lower layer were removed by filtration. The filtrate was combined with chloroform, water, and sodium hydrogen carbonate, stirred for 1 hour, and separated. The lower layer was washed with water two times. After washing, the mixture was separated, and the lower layer was concentrated, combined with methanol, and stirred for 1 hour, followed by crystallization. The precipitated crystals were collected by filtration, dried at 50°C under reduced pressure, and thereby yielded 10.5 g (10.5 mmol; in a yield of 56%) of 3,3'-bis(4-bromophenyl)-5,5',7,7'-tetramethyl-1,1'-biadamantane represented by following Formula (7):

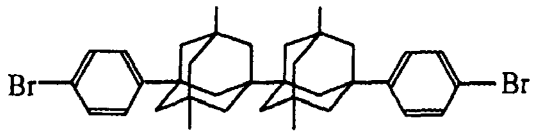

(7)

$^1$H-NMR ($CDCl_3$, 500 MHz) δ: 0.89 (s, 12H), 1.12 (q, 4H), 1.23 (q, 8H), 1.39-1.48 (m, 12H), 7.22 (d, 2H), 7.41 (d, 4H) GC/MS spectrometry: 636 m/z [electron ionization (EI) detection]

Example 3

In a 1-liter four-necked flask in a nitrogen atmosphere were placed 7.34 g (0.044 mol) of iron(III) chloride, 1732 g of benzene, and 260 g (0.0444 mol) of 3,3',5,5'-tetrabromo-1,1'-biadamantane, and the mixture was heated to 70°C, followed by stirring for 4 hours. The reaction mixture after the reaction was cooled to 20°C, combined with 1040 g of water, stirred for 1 hour, and separated. In addition, the mixture was washed with two portions of 1040 g of water, followed by separation. The upper layer was combined with 520 g of methanol and stirred at 5°C for 1 hour. The precipitated crystals were collected by filtration and washed with methanol. Drying at 80°C under reduced pressure gave 178.5 g (0.13 mol; in a yield of 70%) of 3,3',5,5'-tetraphenyl-1,1'-biadamantane.
$^1$H-NMR ($CDCl_3$, 500 MHz) δ: 1.73-1.95 (m, 22H), 2.05 (d, 2H), 2.42 (s, 2H), 7.18 (t, 4H), 7.33 (t, 8H), 7.42 (d, 8H)

In a 1-liter four-necked flask in a nitrogen atmosphere were placed 100 g (0.174 mol) of the above-prepared 3,3',5,5'-tetraphenyl-1,1'-biadamantane, 5.64 g (0.035 mol) of iron(III) chloride, and 1000 g of chloroform, and the mixture was stirred at 20°C for 0.5 hour. Next, the mixture was combined with 124.8 g (0.765 mol) of bromine gradually added, followed by stirring at 40°C for 4 hours. The mixture was further combined with 128.4 g (0.765 mol) of bromine gradually added, followed by stirring at 40°C for 6 hours. The reaction mixture after the reaction was cooled to 5°C, and combined with an aqueous sodium sulfite solution to remove excess of bromine. Next, the mixture was separated, and precipitates were removed by filtration. The filtrate was concentrated, combined with hexane, and stirred at 20°C for 1 hour, followed by crystallization. The precipitated crystals were collected by filtration and washed with hexane. Drying of the obtained crystals at 60°C under reduced pressure gave 108 g (0.121 mol; in a yield of 70%) of 3,3',5,5'-tetrakis(4-bromophenyl)-1,1'-biadamantane represented by following Formula (8):

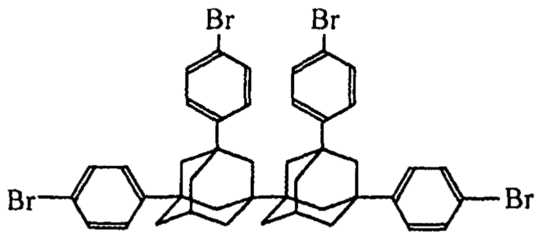

(8)

$^1$H-NMR ($CDCl_3$, 500 MHz) δ: 1.70-2.03 (m, 24H), 2.45 (s, 2H), 7.26 (d, 8H), 7.43 (d, 8H) FAB-MS-spectrometry: 890 m/z

Example 4

In a 1-liter four-necked flask in a nitrogen atmosphere were placed 212.7 g (0.264 mol) of 3,3'-bis(3,5-dibromophenyl)-5,5',7,7'-tetramethyl-1,1'-biadamantane, 14.8 g (0.021 mol) of dichlorobis(triphenylphosphine)palladium, 27.74 g (0.106 mol) of triphenylphosphine, 20.14 g (0.106 mol) of copper iodide, 1470 g of triethylamine, and 630 g of toluene, and the mixture was heated to 70°C and stirred. After the temperature rise, 77.9 g (0.793 mol) of trimethylsilylacetylene was gradually added over 1 hour, followed by stirring at 70°C for 1 hour. In addition, 52 g (0.529 mol) of trimethylsilylacetylene was gradually added over 1 hour, followed by stirring at 70°C for 7 hours. The reaction mixture after the reaction was concentrated, combined with 2000 g of toluene, stirred at 30°C for 1 hour, and insoluble components were separated therefrom by filtration. The filtrate was combined with 180 g of 5 N hydrochloric acid and 500 g of water, the mixture was adjusted to have a pH of 6 and then separated. The upper layer was washed with water two times, separated, concentrated, cooled to 20°C, and combined with 800 g of acetone gradually added, followed by crystallization.

The precipitated crystals were collected by filtration and washed with acetone. To 205 g of the wet crystals was added 1000 g of toluene, the mixture was heated to 50°C to give a solution, and the solution was cooled to 20°C. Next, 2000 g of acetone was gradually added, followed by crystallization. The precipitated crystals were collected by filtration, washed with acetone, dried at 80°C under reduced pressure, and thereby yielded 169 g (0.196 mol; in a yield of 73%) of 3,3'-bis(3,5-trimethylsilylethynylphenyl)-5,5',7,7'-tetramethyl-1,1'-biadamantane represented by following Formula (9):

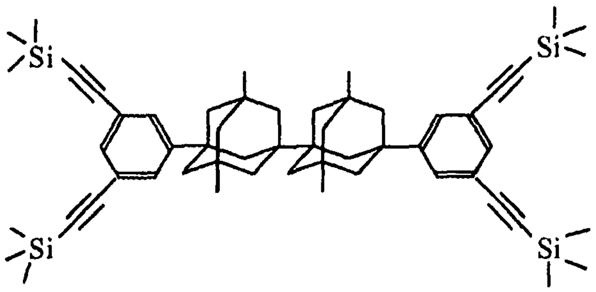

(9)

$^1$H-NMR ($CDCl_3$, 500 MHz) δ: 0.25 (s, 36H), 0.91 (s, 12H), 1.07 (s, 4H), 1.20 (s, 8H), 1.35-1.52 (m, 8H), 1.44 (s, 4H), 7.40 (s, 4H), 7.43 (s, 4H) GC/MS spectrometry: 862.37 m/z [M+]

Example 5

In a 1-liter four-necked flask in a nitrogen atmosphere were placed 10 g (4.6 mmol) of 3,3'-bis(3,5-trimethylsilylethynylphenyl)-5,5',7,7'-tetramethyl-1,1'-biadamantane, 0.64 g (4.6 mmol) of potassium carbonate, 100 g of tetrahydrofuran, and 50 g of methanol, and the mixture was stirred at 30°C for 4 hours. The reaction mixture after the reaction was combined with 50 g of methanol, stirred at 20°C for 1 hour, and the precipitated crystals were collected by filtration. The crystals were dissolved in chloroform with heating, and the solution was washed with water and separated. The lower layer was concentrated, combined with methanol, and the precipitated crystals were collected by filtration and washed with methanol. Drying of the resulting crystals at 50°C under reduced pressure gave 6.3 g (11 mmol; in a yield of 95%) of 3,3'-bis(3,5-ethynylphenyl)-5,5',7,7'-tetramethyl-1,1'-biadamantane represented by following Formula (10):

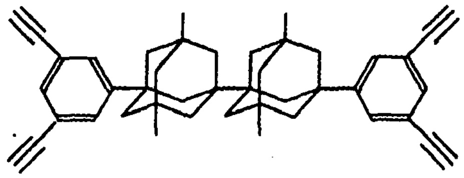

(10)

$^1$H-IVMR ($CDCl_3$, 500 MHz) δ: 0.84 (s, 12H), 1.02 (s, 4H), 1.22 (s, 8H), 1.38-1.51 (m, 12H), 3.01 (s, 4H), 7.45 (s, 2H), 7.47 (s, 4H)

Example 6

In a 1-liter four-necked flask in a nitrogen atmosphere were placed 1.0 g (1.26 mmol) of 3,3'-bis(3,5-dibromophenyl)-5,5',7,7'-tetramethyl-1,1'-biadamantane, 0.071 g (0.1 mmol) of dichlorobis(triphenylphosphine)palladium, 0.132 g (0.5 mmol) of triphenylphosphine, 0.096 g (0.5 mmol) of copper iodide, 11 g of triethylamine, and 5 g of toluene, and the mixture was heated to 70°C and stirred. After the temperature rise, 1.03 g (10.0 mmol) of phenylacetylene was gradually added over 1.5 hour, followed by stirring with heating at 70°C for 12 hours. The reaction mixture after the reaction was concentrated, combined with 64 g of toluene, stirred at 50°C for 1 hour, and insoluble components were separated therefrom by filtration. The filtrate was combined with 10 g of 5 N hydrochloric acid and separated. The upper layer was washed with water two times, concentrated, cooled, and the precipitated crystals were collected by filtration and washed with acetone. Drying of the obtained wet crystals at 80°C under reduced pressure gave 0.72 g (0.82 mmol; in a yield of 65%) of 3,3'-bis(3,5-phenylethynylphenyl)-5,5',7,7'-tetramethyl-1,1'-biadamantane represented by following Formula (11):

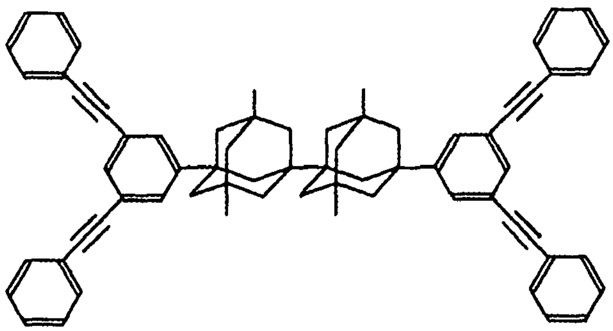

(11)

DI/MS-spectrometry: 879.25 m/z [electron ionization (EI) detection]

Examples 1 to 6 demonstrate that processes according to embodiments of the present invention yield halophenylbiadamantane derivatives and ethynylphenylbiadamantane derivatives according to embodiments of the present invention in high yields.

While there have been described what are at present considered to be the preferred embodiments of the present invention, it should be understood by those skilled in the art that various modifications, combinations, subcombinations, and alterations may occur depending on design requirements and other factors insofar as they are within the spirit and scope of the appended claims or the equivalents thereof.

## Claims

1. A halophenylbiadamantane derivative represented by following Formula (1):

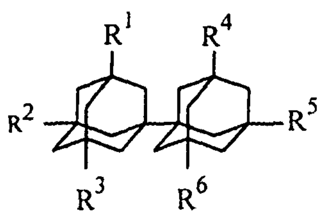

(1)

wherein $R^1$, $R^2$, $R^3$, $R^4$, $R^5$, and $R^6$ are the same as or different from one another and each represent a hydrogen atom, a halo-substituted or unsubstituted alkyl or cycloalkyl group having about six or less carbon atom(s), a substituted or unsubstituted phenyl group, a carboxyl group, a substituted or unsubstituted carbamoyl group, a substituted oxycarbonyl group, a substituted or unsubstituted amino group, a halogen atom, or a hydroxyl group,
with the proviso that at least one of $R^1$ to $R^6$ represents a substituent represented by following Formula (2):

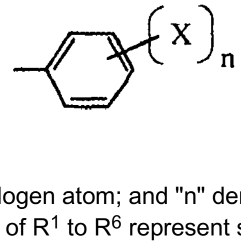

(2)

wherein X represents a halogen atom; and "n" denotes a natural number of 1 to 5,
wherein when two or more of $R^1$ to $R^6$ represent substituents of Formula (2), these substituents may be the same as or different from one another,
wherein the benzene ring in Formula (2) may further have an alkyl or cycloalkyl group having about six or less carbon atoms in addition to the halogen atom(s), and
wherein when only one of $R^1$ to $R^6$ is a substituent of Formula (2), then at least one of $R^1$ to $R^6$ is a halo-substituted

or unsubstituted alkyl or cycloalkyl group having about six or less carbon atom(s) or "n" is a natural number of 2 or more.

2. An ethynylphenylbiadamantane derivative represented by following Formula (3):

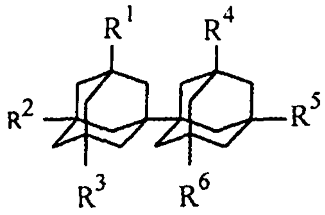

(3)

wherein $R^1$, $R^2$, $R^3$, $R^4$, $R^5$, and $R^6$ are the same as or different from one another and each represent a hydrogen atom, a halo-substituted or unsubstituted alkyl or cycloalkyl group having about six or less carbon atom(s), a substituted or unsubstituted phenyl group, a carboxyl group, a substituted or unsubstituted carbamoyl group, a substituted oxycarbonyl group, a substituted or unsubstituted amino group, a halogen atom, or a hydroxyl group,
with the proviso that at least one of $R^1$ to $R^6$ represents a substituent represented by following Formula (4) :

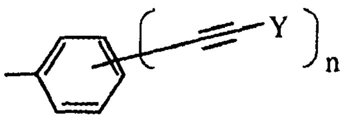

(4)

wherein Y represents a hydrogen atom, an alkyl group having about one to about six carbon atoms, a phenyl group, or a tri-substituted silyl group; and "n" denotes a natural number of 1 to 5,
wherein when two or more of $R^1$ to $R^6$ represent substituents of Formula (4), these substituents may be the same as or different from one another, and
wherein when "n" is 1 in all the substituent(s) of Formula (4) as $R^1$ to $R^6$, then at least one of $R^1$ to $R^6$ is a halo-substituted or unsubstituted alkyl or cycloalkyl group having about six or less carbon atom(s).

3. A process for the preparation of ethynylphenylbiadamantane derivatives, the process comprising the step of reacting a halophenyladamantane derivative with a terminal alkyne to give an ethynylphenylbiadamantane derivative,
the halophenyladamantane derivative represented by following Formula (1a):

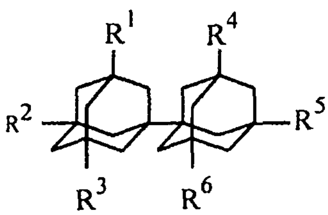

(1 a)

wherein $R^1$, $R^2$, $R^3$, $R^4$, $R^5$, and $R^6$ are the same as or different from one another and each represent a hydrogen atom, a halo-substituted or unsubstituted alkyl or cycloalkyl group having about six or less carbon atom(s), a substituted or unsubstituted phenyl group, a carboxyl group, a substituted or unsubstituted carbamoyl group, a substituted oxycarbonyl group, a substituted or unsubstituted amino group, a halogen atom, or a hydroxyl group,
with the proviso that at least one of $R^1$ to $R^6$ represents a substituent represented by following Formula (2):

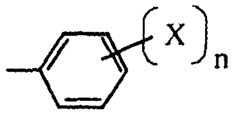

(2)

wherein X represents a halogen atom; and "n" denotes a natural number of 1 to 5,
wherein, when two or more of $R^1$ to $R^6$ represent substituents of Formula (2), these substituents may be the same as or different from one another,
wherein the benzene ring in Formula (2) may further have an alkyl or cycloalkyl group having about six or less carbon atoms in addition to the halogen atom(s), and
wherein when "n" is 1 in all the substituent(s) of Formula (2) as $R^1$ to $R^6$, then at least one of $R^1$ to $R^6$ is a halo-substituted or unsubstituted alkyl or cycloalkyl group having about six or less carbon atom(s),
the terminal alykyne represented by following Formula (5):

$$R'-C\equiv C-Y \qquad (5)$$

wherein Y represents a hydrogen atom, an alkyl group having about one to about six carbon atoms, a phenyl group, or a tri-substituted silyl group; and R' represents a hydrogen atom or a metal atom, and
the ethynylphenylbiadamantane derivative represented by following Formula (3):

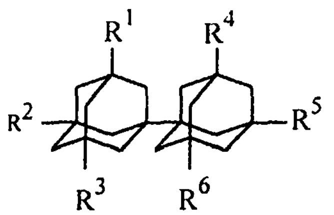

(3)

wherein $R^1$, $R^2$, $R^3$, $R^4$, $R^5$, and $R^6$ are the same as or different from one another and each represent a hydrogen atom, a halo-substituted or unsubstituted alkyl or cycloalkyl group having about six or less carbon atom(s), a substituted or unsubstituted phenyl group, a carboxyl group, a substituted or unsubstituted carbamoyl group, a substituted oxycarbonyl group, a substituted or unsubstituted amino group, a halogen atom, or a hydroxyl group,
with the proviso that at least one of $R^1$ to $R^6$ represents a substituent represented by following Formula (4):

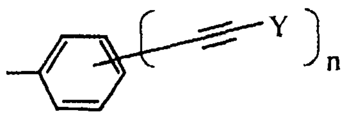

(4)

wherein Y represents a hydrogen atom, an alkyl group having about one to about six carbon atoms, a phenyl group, or a tri-substituted silyl group; and "n" denotes a natural number of 1 to 5,
wherein when two or more of $R^1$ to $R^6$ represent substituents of Formula (4), these substituents may be the same as or different from one another, wherein when "n" is 1 in all the substituent(s) of Formula (4) as $R^1$ to $R^6$, then at least one of $R^1$ to $R^6$ is a halo-substituted or unsubstituted alkyl or cycloalkyl group having about six or less carbon atom(s).

**4.** A process for the preparation of ethynylphenylbiadamantane derivatives, the process comprising the step of reacting an ethynylphenylbiadamantane derivative (3a) with an alcohol compound to give a ethynylphenylbiadamantane derivative (3b),

the ethynylphenylbiadamantane derivative (3a) represented by following Formula (3a):

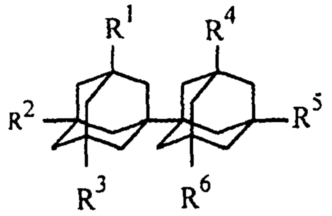

(3a)

wherein $R^1$, $R^2$, $R^3$, $R^4$, $R^5$, and $R^6$ are the same as or different from one another and each represent a hydrogen atom, a halo-substituted or unsubstituted alkyl or cycloalkyl group having about six or less carbon atom(s), a substituted or unsubstituted phenyl group, a carboxyl group, a substituted or unsubstituted carbamoyl group, a substituted oxycarbonyl group, a substituted or unsubstituted amino group, a halogen atom, or a hydroxyl group,
with the proviso that at least one of $R^1$ to $R^6$ represents a substituent represented by following Formula (4a):

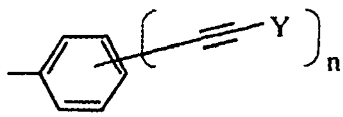

(4a)

wherein Y represents a tri-substituted silyl group; and "n" denotes a natural number of 1 to 5,
wherein when two or more of $R^1$ to $R^6$ represent substituents of Formula (4a), these substituents may be the same as or different from one another, wherein when "n" is 1 in all the substituent(s) of Formula (4a) as $R^1$ to $R^6$, then at least one of $R^1$ to $R^6$ is a halo-substituted or unsubstituted alkyl or cycloalkyl group having about six or less carbon atom(s),
the ethynylphenylbiadamantane derivative (3b) represented by following Formula (3b):

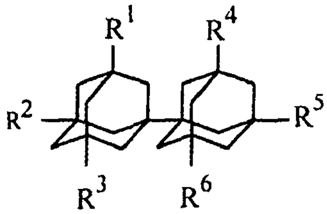

(3b)

wherein $R^1$, $R^2$, $R^3$, $R^4$, $R^5$, and $R^6$ are the same as or different from one another and each represent a hydrogen atom, a halo-substituted or unsubstituted alkyl or cycloalkyl group having about six or less carbon atom(s), a substituted or unsubstituted phenyl group, a carboxyl group, a substituted or unsubstituted carbamoyl group, a substituted oxycarbonyl group, a substituted or unsubstituted amino group, a halogen atom, or a hydroxyl group,
with the proviso that at least one of $R^1$ to $R^6$ represents a substituent represented by following Formula (4b) :

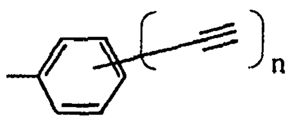

(4b)

wherein "n" denotes a natural number of 1 to 5,
wherein when two or more of $R^1$ to $R^6$ represent substituents of Formula (4b), these substituents may be the same as or different from one another, wherein when "n" is 1 in all the substituents(s) represented by Formula (4b) as $R^1$

to $R^6$, then at least one of $R^1$ to $R^6$ is a halo-substituted or unsubstituted alkyl or cycloalkyl group having about six or less carbon atom(s).

Europäisches Patentamt
European Patent Office
Office européen des brevets

# EUROPEAN SEARCH REPORT

Application Number
EP 08 01 1056

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/034902 A (SUMITOMO BAKELITE CO [JP]; ENOKI TAKASHI [JP]; IZUMI ATSUSHI [JP]; OKI) 29 March 2007 (2007-03-29) * page 44, lines 11,12 * ----- | 2 | INV. C07C13/615 C07C25/02 |
| P,X | WO 2007/111168 A (SUMITOMO BAKELITE CO [JP]; FUJITA KAZUYOSHI [JP]; IZUMI ATSUSHI [JP];) 4 October 2007 (2007-10-04) * paragraphs [0183], [0185], [0187] * ----- | 1-4 | |

TECHNICAL FIELDS SEARCHED (IPC)
C07C

The present search report has been drawn up for all claims

| Place of search | Date of completion of the search | Examiner |
|---|---|---|
| The Hague | 17 November 2008 | De Borggraeve, Wim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 08 01 1056

This annex lists the patent family members relating to the patent documents cited in the above-mentioned European search report.
The members are as contained in the European Patent Office EDP file on
The European Patent Office is in no way liable for these particulars which are merely given for the purpose of information.

17-11-2008

| Patent document cited in search report | Publication date | Patent family member(s) | Publication date |
|---|---|---|---|
| WO 2007034902 A | 29-03-2007 | EP 1953181 A1 | 06-08-2008 |
| | | KR 20080053382 A | 12-06-2008 |
| WO 2007111168 A | 04-10-2007 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003292878 A
- JP 2006257212 A
- JP 2005516382 A
- JP 2006096988 A